# EUROPÄISCHE PATENTSCHRIFT

(11) **EP 1 337 231 B1**
(45) Veröffentlichungstag und Bekanntmachung des Hinweises auf die Patenterteilung: **08.08.2007**
(21) Anmeldenummer: 01998313.9
(22) Anmeldetag: 21.11.2001
(51) Int. Cl.: A61K 8/04, A61K 8/34, A61K 8/46, A61K 8/60, A61K 8/92, A61Q 5/02, A61Q 5/12

(54) **VERWENDUNG VON NANOPARTIKULÄREN WACHSEN IN DER HAARKOSMETIK**
USE OF NANOPARTICULATE WAX IN HAIR COSMETICS
UTILISATION DE CIRES NANOPARTICULAIRES EN COSMETIQUE CAPILLAIRE

(30) Priorität: 01.12.2000 DE 10059670
(43) Veröffentlichungstag der Anmeldung: 27.08.2003
(73) Patentinhaber: Henkel Kommanditgesellschaft auf Aktien, 40589 Düsseldorf (DE)
(72) Erfinder: HUNDEIKER, Claudia, 40549 Düsseldorf (DE); KROPF, Christian, 40721 Hilden (DE); SCHRÖDER, Christine, 40593 Düsseldorf (DE); SCHULZE ZUR WIESCHE, Erik, 20251 Hamburg (DE); HOLLENBROCK, Martina, 40723 Hilden (DE); FÖRSTER, Thomas, 40699 Erkrath (DE); DOLHAINE, Hans, 41352 Korschenbroich (DE)
(86) Internationale Anmeldenummer: PCT/EP2001/013480
(87) Internationale Veröffentlichungsnummer: WO 2002/043671

(56) Entgegenhaltungen:
- DE-A- 4 441 029
- DE-A- 19 710 149
- DE-A- 19 837 191
- DE-A- 19 911 041
- DE-A- 19 958 521
- FR-A- 2 666 015

## Beschreibung

Die Erfindung betrifft die Verwendung nanopartikulärer Wachse, wobei die Wachse einen Schmelzpunkt von mindestens 40°C haben und wobei der mittlere Teilchendurchmesser im Bereich von 5 bis 200 nm liegt, zur Pflege von Keratinfasern, insbesondere menschlichen Haaren. Die Wachse üben auf die Keratinfasern eine konditionierende Wirkung aus und verbessern insbesondere die Naßkämmbarkeit und den Griff.

Die Reinigung und Pflege der Haare ist ein wichtiger Bestandteil der menschlichen Körperpflege. Sowohl die Reinigung und Pflege der Haare beispielsweise mit Shampoos als auch die dekorative Gestaltung der Frisur beispielsweise durch Färben oder Dauerwellen sind Eingriffe, die die natürliche Struktur und die Eigenschaften der Haare beeinflussen. So können anschließend an eine solche Behandlung beispielsweise die Naß- und Trockenkämmbarkeit des Haares, der Halt und die Fülle des Haares unbefriedigend sein. Weiterhin können die Haare einen erhöhten Spliß aufweisen oder aufgrund elektrostatischer Aufladung "fliegen".

Es ist daher seit langem üblich, die Haare einer speziellen Nachbehandlung zu unterziehen. Dabei werden, üblicherweise in Form einer Spülung, die Haare mit speziellen Wirkstoffen, beispielsweise quaternären Ammoniumsalzen oder speziellen Polymeren, behandelt. Durch diese Behandlung werden je nach Formulierung Kämmbarkeit, Halt und Fülle der Haare verbessert und die Splißrate verringert. Es besteht weiterhin ein Bedarf an Wirkstoffen, die sich direkt in die verschiedenen Haarbehandlungsmittel einarbeiten lassen und somit den zusätzlichen Nachbehandlungsschritt überflüssig machen.

Zusätze von kationischen Polymeren zu Haarbehandlungsmitteln führen in der Regel zu einer verbesserten Naß- und Trockenkämmbarkeit; Zusätze von amphoteren Polymeren zu einer stark verbesserten Naßkämmbarkeit, während die Trockenkämmbarkeit meist nur wenig beeinflußt wird.

Während die Verbesserung der Naßkämmbarkeit, d.h. eine Erniedrigung der Naßkämmarbeit, in allen Fällen gewünscht wird, sind die Verhältnisse bei der Trockenkämmbarkeit komplizierter. Niedrige Kämmarbeits-Werte charakterisieren zwar eine Verbesserung der Kämmbarkeit; wird die Kämmarbeit aber zu sehr erniedrigt, so verliert das Haar Fülle und Halt, so daß sich im Extremfall bestimmte Frisuren nicht mehr aufbauen lassen. Daher kann, vor allem bei komplexeren Frisuren, eine in bestimmten Grenzen erhöhte Trockenkämmarbeit durchaus erwünscht sein, um den Halt der Frisur zu verbessern. Diese ist in vielen Fällen aber mit einer erhöhten elektrostatischen Aufladung der Haare verbunden, was zum unerwünschten Phänomen des "Fliegens" der Haare führt. Eine weiterer wichtiger Parameter ist der sogenannte Griff der Haare, welcher als die subjektive Empfindung beim manuellen Kontakt mit dem Haar beschrieben werden kann.

Bei der kosmetischen Reinigung und Pflege der Haare kommen den Parametern Naßkämmbarkeit, Trockenkämmbarkeit und Griff der Haare eine besondere Bedeutung für die Produktauswahl und -akzeptanz durch den Anwender zu. Neben der Ausprägung der einzelnen Parameter, welche im Sinne der vorliegenden Erfindung unter dem Begriff "Konditionierwirkung" zusammengefaßt und verstanden werden sollen, ist auch eine ausgewogene Relation der Parameter untereinander wesentlich.

Obwohl aus dem Stand der Technik bereits zahlreiche Lösungsvorschläge zur Verbesserung der Konditionierwirkung haarkosmetischer Produkte vorliegen, wie z. B. diverse quaternäre Stickstoffverbindungen sowie Silicone, vermögen diese nicht in allen Fällen zu befriedigen.

So ist bekannt, daß es bei quaternären Stickstoffverbindungen bei wiederholter Anwendung zu einem Additionseffekt kommen kann, bei dem durch die zunehmende Anlagerung der kationischen Verbindungen das Haar schwer und strähnig wird.

Es besteht somit nach wie vor ein Bedarf an verbesserten Wirkstoffen zur Erzielung einer Konditionierwirkung an Haaren, die darüber hinaus gut in bekannte Haarreinigungs- und Pflegemittel eingebaut werden können.

Überraschenderweise wurde gefunden, daß nanopartikuläre Wachse mit einem Schmelzpunkt von mindestens 40°C und einem mittleren Teilchendurchmesser des Wachses im Bereich von 5 bis 200 nm in besonderem Maße zur Konditionierung von Keratinfasern, insbesondere menschlichen Haaren geeignet sind und einen erheblichen Beitrag zur Lösung der aus dem Stand der Technik bekannten Problematik leisten können.

Die Verwendung von Wachsen in der Haarkosmetik ist lange bekannt. Wegen der Probleme bei der Einarbeitung von Wachsen in wäßrige Formulierungen gibt es bereits seit einigen Jahren Bemühungen, Wachse in Form von feinstteiligen Dispersionen einzusetzen.

So beschreibt die europäische Patentschrift 0 394 078 kosmetische Zusammensetzungen zur Behandlung von Haaren, welche eine Wachs-Mikrodispersion enthalten. Die Zusammensetzungen sind nicht schäumend und frei von kationischen oberflächenaktiven Stoffen. Beschrieben werden in diesem Dokument vor bzw. nach dem Schamponieren der Haare aufzubringende Zubereitungen sowie Frisierlotionen und -gele, welche auf dem gesäuberten und trockenen Haar aufzubringen sind. Sie verleihen der Frisur Volumen und den Haaren mehr Fülle und Glanz, ohne ihnen ein fettiges Aussehen zu geben. Weiterhin sollen die Haare gegenüber UV-Strahlung geschützt werden. Daß nanopartikuläre Wachsdispersionen die Konditionierwirkung haarkosmetischer Zusammensetzungen im vorstehend definierten Sinn verbessern können, ist aus diesem Dokument jedoch nicht bekannt. Ebenso wenig gibt es dem Fachmann Hinweise auf schäumende haarkosmetische Zusammensetzungen wie z. B. Haarshampoos, welche nanopartikuläre Wachse enthalten und dadurch eine verbesserte Konditionierwirkung aufweisen. Die Verwendung von Wachsen in schäumenden Formulierungen wird bekanntermaßen dadurch limitiert, daß stark lipophile Stoffe wie Wachse schaumdämpfend wirken. Dadurch lassen sich Wachse für viele Anwendungen nicht im gewünschten Ausmaß in schäumende Formulierungen einarbeiten.

DE 44 41 029A1 offenbart eine wässrige Dispersion eines nanopartikulären Wachses (Cetylpalmitat) mit einem mittleren Teilchendurchmesser von 150 nm, wobei das Wachs mit Behenylalkoholpoly(10) glycolether, Glycerinmono- und -distearat und dem kationischen Tensid Cetyltrimethylammoniumchlorid als Oberflächenmodifikationsmittel stabilisiert ist.
DE 197 10 149 A1 offenbart wässrige Dispersionen nanopartikulärer Wachse (Cetylpalmitat + hydriertes Rizinusöl) mit einem mittleren Teilchendurchmesser von 172 bzw. 199 nm, wobei die Wachse mit Behenylalkoholpoly(10)glycolether allein oder zusammen mit Glycerinmonopalmitat als Oberflächenmodifikationsrnittel stabilisiert sind.
DE 198 37 191 A1 offenbart wässrige Dispersionen nanopartikulärer Wachse (Cetylpalmitat + hydriertes Rizinusöl, Paraffinwachs) mit einem mittleren Teilchendurchmesser von 180- 480 nm, wobei die Wachse mit Behenylalkoholpoly(10)glycolether allein oder zusammen mit Glycerinmonopalmitat als Oberflächenmodifkationsmittel stabilisiert sind.
FR 2 666 015 A1 offenbart kosmetische und/oder dermopharrnazeutische Zusammensetzungen, die eine wässrige Dispersion nanopartikulärer Wachse mit einer Teilchengröße kleiner 500 nm, die mit diversen anionischen oder nicht-ionischen Emulgatoren stabilisiert sind, sowie deren Verwendung zur Verabreichung von Retinoiden in die Haut.
EP 506 197 A1 offenbart wässrige Dispersionen nanopartikulärer Wachse, die mit diversen Emulgatoren (kationische, anionische und amphotere Emulgatoren, weiterhin Polyoxyethylenpolyoxypropylenalkylether, Polyoxyethylenfettsäureester, Polyoxyethylensorbitanfettsäureester, Polyoxyethylenalkylether, Sorbitanester, Sucroseester, Lecithine, Siliconemulgatoren, Betaine und Polyglycerylfettsäureester) stabilisiert sind, sowie deren Verwendung in topischen Zusammensetzungen.

Gegenstand der Erfindung ist ein nanopartikuläres Wachs, wobei das Wachs einen Schmelzpunkt von mindestens 40°C hat, der mittlere Teilchendurchmesser des Wachses im Bereich von 5 bis 200 nm liegt, das nanopartikuläre Wachs von mindestens einem Oberflächenmodifikationsmittel, das ein Alkylpolyglycosid ist, umhüllt ist und für die Herstellung des nanopartikulären Wachses das Wachs und das Oberflächenmodifikationsmittel im Gewichtsverhältnis 1 : 2 bis 2 : 1 eingesetzt werden, ausgenommen
a) ein nanoskaliges Wachs mit einer Partikelgröße von 70 nm, hergestellt aus 10 Gewichtsteilen Lanolin, 10 Gewichtsteilen des Handelsproduktes Texapon N 70 (entsprechend 6,8 - 7,3 Gewichtsteilen Natriumlaureth-2-sulfat) und 10 Gewichtsteilen des Handelsproduktes Plantacare 818 (entsprechend 5,1 - 5,3 Gewichtsteilen Coco Glucosides);
b) ein nanoskaliges Wachs mit einer Partikelgröße von 100 nm, hergestellt aus 15 Gewichtsteilen Lanolin und 15 Gewichtsteilen des Handelsproduktes Plantacare 818 (entsprechend 7,65 - 7,95 Gewichtsteilen Coco Glucosides).

Ein weiterer Gegenstand der Erfindung ist eine kosmetische Zubereitung, enthaltend ein nanopartikuläres Wachs, wobei das Wachs einen Schmelzpunkt von mindestens 40°C hat, der mittlere Teilchendurchmesser des Wachses im Bereich von 5 bis 200 nm liegt, das nanopartikuläre Wachs von mindestens einem Oberflächenmodifikationsmittel, das ein Alkylpolyglycosid ist, umhüllt ist und für die Herstellung des nanopartikulären Wachses das Wachs und das Oberflächenmodifikationsmittel im Gewichtsverhältnis 1 : 2 bis 2: 1 eingesetzt werden, ausgenommen
a) ein nanoskaliges Wachs mit einer Partikelgröße von 70 nm, hergestellt aus 10 Gewichtsteilen Lanolin, 10 Gewichtsteilen des Handelsproduktes Texapon N 70 (entsprechend 6,8 - 7,3 Gewichtsteilen Natriumlaureth-2-sulfat) und 10 Gewichtsteilen des Handelsproduktes Plantacare 818 (entsprechend 5,1 - 5,3 Gewichtsteilen Coco Glucosides);
b) ein nanoskaliges Wachs mit einer Partikelgröße von 100 nm, hergestellt aus 15 Gewichtsteilen Lanolin und 15 Gewichtsteilen des Handelsproduktes Plantacare 818 (entsprechend 7,65 - 7,95 Gewichtsteilen Coco Glucosides).

Ein weiterer Gegenstand der Erfindung ist die Verwendung eines nanopartikulären Wachses, wobei das Wachs einen Schmelzpunkt von mindestens 40°C hat, der mittlere Teilchendurchmesser des Wachses im Bereich von 5 bis 200 nm liegt, das nanopartikuläre Wachs von mindestens einem Oberflächenmodifikationsmittel, das ein Alkylpolyglycosid ist, umhüllt ist und für die Herstellung des nanopartikulären Wachses das Wachs und das Oberflächenmodifikationsmittel im Gewichtsverhältnis 1 : 2 bis 2: 1 eingesetzt werden, zur Behandlung und/oder zur Pflege von Keratinfasern, insbesondere menschlichen Haaren, ausgenommen
a) ein nanoskaliges Wachs mit einer Partikelgröße von 70 nm, hergestellt aus 10 Gewichtsteilen Lanolin, 10 Gewichtsteilen des Handelsproduktes Texapon N 70 (entsprechend 6,8 - 7,3 Gewichtsteilen Natriumlaureth-2-sulfat) und 10 Gewichtsteilen des Handelsproduktes Plantacare 818 (entsprechend 5,1 - 5,3 Gewichtsteilen Coco Glucosides);
b) ein nanoskaliges Wachs mit einer Partikelgröße von 100 nm, hergestellt aus 15 Gewichtsteilen Lanolin und 15 Gewichtsteilen des Handelsproduktes Plantacare 818 (entsprechend 7,65 - 7,95 Gewichtsteilen Coco Glucosides).

Die Größenangaben sind zu verstehen als Durchmesser in Richtung der größten Längenausdehnung der Teilchen. Bei der Herstellung der feinteiligen Partikel erhält man stets Teilchen mit einer Größe, die einer Verteilungskurve folgt. Zur experimentellen Bestimmung der Teilchengröße kann beispielsweise die dem Fachmann bekannte Methode der dynamischen Lichtstreuung angewandt werden.

Unter Wachsen sind im Sinne der Erfindung natürliche oder synthetische Stoffe zu verstehen, welche bei 20°C knetbar, fest bis brüchig hart, grob bis feinkristallin, durchscheinend bis opak, jedoch nicht glasartig sind, oberhalb von ca. 40°C unzersetzt schmelzen und schon wenig oberhalb des Schmelzpunktes niedrigviskos und nicht fadenziehend sind. Die im Sinne der Erfindung einzusetzenden Wachse unterscheiden sich beispielsweise von Harzen dadurch, daß sie in der Regel etwa zwischen und 50 und 90°C, in Ausnahmefällen auch bis zu 200°C, in den schmelzflüssigen, niedrigviskosen Zustand übergehen und praktisch frei von aschebildenden Verbindungen sind. Nach ihrer Herkunft teilt man die Wachse in die folgenden drei Gruppen ein: natürliche Wachse pflanzlichen oder tierischen Ursprungs, wie z.B. Candelillawachs, Carnaubawachs,Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse oder hydriertes Rizinusöl sowie synthetische Wachse, wie z.B. Polyalkylenwachse und Polyethylenglycolwachse. Ein erfindungsgemäß besonders bevorzugtes Wachs ist hydriertes Rizinusöl.

Ebenso sind zu den Wachsen im Sinne der Erfindung die höheren Fettalkohole sowie die Wachsester zu zählen, sofern sie einen Schmelzpunkt von mindestens 40°C haben.

Unter höheren Fettalkoholen sind primäre aliphatische Alkohole der Formel (1) zu verstehen,

R¹OH (I)

in der R¹ für einen aliphatischen, linearen oder verzweigten Kohlenwasserstoffrest mit 14 bis 40 Kohlenstoffatomen und 0, 1, 2 oder 3 Doppelbindungen steht. Typische Beispiele sind Myristylalkohol, Cetylalkohol, Palmoleylalkohol, Stearylalkohol, lsostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol, Brassidylalkohol, Lignocerylalkohol, Cerylalkohol, Myricylalkohol, Melissylalkohol sowie deren Mischungen. Die Fettalkohole können pflanzlichen, tierischen oder synthetischen Ursprungs sein. Erfindungsgemäß verwendbare synthetische Fettalkohol-Gemische werden z. B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen erhalten. Bevorzugt sind höhere Fettalkohole pflanzlichen Ursprungs, besonders bevorzugt solche mit 16 bis 18 Kohlenstoffatomen. Weiterhin bevorzugt ist Cetylstearylalkohol, worunter Gemische aus etwa gleichen Teilen Cetyl- und Stearylalkohol zu verstehen sind.

Unter Wachsestern sind Stoffe zu verstehen, die der Formel (II) folgen,

R¹COO-R² (II)

in der R¹CO für einen linearen oder verzweigten Acylrest mit 6 bis 22 Kohlenstoffatomen, mit 0 bis 3 Doppelbindungen und mit 0 bis 3 Hydroxy-Substituenten steht und R² für einen linearen oder verzweigten Alkyl- und/oder Alkenylrest mit 6 bis 22 Kohlenstoffatomen, mit der Maßgabe, daß die Anzahl der Kohlenstoffatome im Ester mindestens 20 beträgt. Typische Beispiele hierfür sind Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, lsostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat, Erucylerucat sowie Ester der Ricinolsäure. Erfindungsgemäß bevorzugt sind Wachsester, in welchen R¹CO für einen linearen Acylrest mit 16 oder 18 Kohlenstoffatomen steht.

Die nanopartikulären Wachspartikel im Sinne der vorliegenden Erfindung sind vorzugsweise von mindestens einem Oberflächenmodifikationsmittel ummantelt, wobei es sich im Sinne der Erfindung versteht, daß auch die ummantelten Wachs-Nanopartikel einen Schmelzpunkt von mindestens ca. 40°C haben.

Unter Oberflächenmodifikationsmitteln sind Stoffe zu verstehen, welche der Oberfläche der Nanopartikel physikalisch anhaften, mit diesen jedoch vorzugsweise nicht chemisch reagieren. Die einzelnen an der Oberfläche adsorbierten Moleküle der Oberflächenmodifikationsmittel sind im wesentlichen frei von intermolekularen Bindungen untereinander. Unter Oberflächenmodifikationsmitteln sind insbesondere Dispergiermittel zu verstehen. Dispergiermittel sind dem Fachmann beispielsweise auch unter den Begriffen Emulgatoren, Schutzkolloide, Netzmittel und Detergentien bekannt.

Das Wachs wird von mindestens einem Oberflächenmodifikationsmittel, das ein Alkylpolyglucosid ist, umhüllt.

Als zusätzliche Oberflächenmodifikationsmittel kommen beispielsweise Emulgatoren vom Typ der nichtionischen Tenside aus mindestens einer der folgenden Gruppen in Frage:
(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester, wie z.B. Polyglycerinpolyricinoleat, Polyglycerinpoly-12-hydroxystearat oder Polyglycerindimerat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipenta-erythrit, Zuckeralkohole (z.B. Sorbit), Alkylglucoside (z.B. Methylglucosid, Butylglucosid, Lauryl-glucosid) sowie Polyglucoside (z.B. Cellulose);
(9) Mono-, Di- und Trialkylphosphate sowie Mono-, Di- und/oder Tri-PEG-alkylphosphate und deren Salze;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß DE-PS 1165574 und/oder Mischester von Fettsäuren mit 6 bis 22 Kohlenstoffatomen, Methylglucose und Polyolen, vorzugsweise Glycerin oder Polyglycerin sowie
(13) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht.

C_{8/18}-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Typische Beispiele für anionische Tenside und Emulgatoren sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Alkylethersulfate wie beispielsweise Fettalkoholethersulfate, Glycerinethersulfate, Hydroxymischethersulfate, Monoglycerid(ether)sulfate, Fettsäureamid(ether)sulfate, Mono- und Dialkyl-sulfosuccinate, Mono- und Dialkylsulfo-succinamate, Sulfotriglyceride, Amidseifen, Ethercarbonsäuren und deren Salze, Fettsäureisethionate, Fettsäuresarcosinate, Fettsäuretauride, N-Acylaminosäuren wie beispielsweise Acylglutamate und Acylaspartate, Alkyloligoglucosidsulfate, Proteinfettsäurekondensate (insbesondere pflanzliche Produkte auf Weizenbasis), und Alkyl(ether)-phosphate. Sofern die anionischen Tenside Polyglycoletherketten enthalten, können diese eine konventionelle, vorzugsweise jedoch eine eingeengte Homologenverteilung aufweisen.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammonium-glycinat, N-Acylamino-propyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung Cocamidopropyl Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methyl-quaternierte Difettsäuretriethanolaminester-Salze, besonders bevorzugt sind.

Als Oberflächenmodifikationsmittel geeignete Schutzkolloide sind z.B. natürliche wasserlösliche Polymere wie z. B. Gelatine, Casein, Gummi arabicum, Lysalbinsäure, Stärke, Albumin, Alginsäure sowie deren Alkali- und Erdalkalimetallsalze, wasserlösliche Derivate von wasserunlöslichen polymeren Naturstoffen wie z. B. Celluloseether wie Methylcellulose, Hydroxyethylcellulose, Carboxymethylcellulose oder modifizierte Carboxymethyl-cellulose, Hydroxyethyl-Stärke oder Hydroxypropyl-Guar, sowie synthetische wasserlösliche Polymere, wie z. B. Polyvinylalkohole, Polyvinylbutyrale, Polyvinylpyrrolidone, Polyalkylenglycole, Polyasparaginsäuren und Polyacrylate.

Als Oberflächenmodifikationsmittel bevorzugt sind Gelatine, Stärke, Dextrin, Dextran, Pektin, Gummi arabicum, Kasein, Polyvinylalkohole, Polyvinylbutyrale, Polyvinylpyrrolidone, Polyalkylenglycole, Methylcellulose, Carboxymethylcellulose, Hydroxypropylcellulose, Fettalkoholpolyglykolether, Fettalkoholpolyglycoside, Fettsäurealkanolamide, Glycerolester und Sorbitanester. Besonders bevorzugt sind Alkylpolyglycoside, in welchen der Alkylrest zwischen 12 und 16 Kohlenstoffatome enthält.

Die Menge des Oberflächenmodifikationsmittels in Bezug auf das Wachs entspricht mindestens der Menge, die erforderlich ist, um eine stabile Dispersion an Wachs-Nanopartikeln zu erhalten. Diese minimale Menge kann jeweils durch einfache Routineversuche ermittelt werden.

Für die Herstellung des nanopartikulären Wachses werden das Wachs und das Oberflächenmodifikationsmittel im Gewichtsverhältnis 1: 2 bis 2 : 1 eingesetzt. Anstelle der angegebenen Mengenanteile des Oberflächenmodifikationsmittels kann auch in gleicher Menge ein Gemisch aus zwei oder mehr Oberflächenmodifikationsmitteln eingesetzt werden.

Die erfindungsgemäß verwendeten nanopartikulären Wachsteilchen können nach unterschiedlichen Verfahren hergestellt werden.

Bei der Herstellung nach dem Evaporationsverfahren wird das Wachs zunächst in einem geeigneten organischen Lösungsmittel, wie z. B. einem Alkan, verzehrbaren Öl, Alkohol, Ether, Ester, Keton oder Acetal gelöst. Anschließend wird diese Lösung derart in ein Nicht-Lösungsmittel für das Wachs, vorzugsweise Wasser, einen niedrigen Alkohol oder Gemische aus mehreren Nicht-Lösungsmitteln, gegebenenfalls in Gegenwart eines darin gelösten Oberflächenmodifikationsmittels, gegeben, daß es durch die Vermischung von organischem Lösungsmittel und Nicht-Lösungsmittel zu einer Ausfällung des nanopartikulären Wachses kommt. Diese Vermischung erfolgt bevorzugt auf eine Weise, bei der das organische Lösungsmittel verdampft und weitgehend aus dem System abgetrennt wird. Das Lösungsmittel kann aber auch nach der Vermischung z. B. durch Destillation entfernt werden. Anstelle der Lösung aus Wachs und organischem Lösungsmittel kann auch eine O/W-Emulsion oder O/W-Mikroemulsion eingesetzt werden, in der die Lösung die Ölkomponente bildet. Als oberflächenaktive Verbindungen können die bereits eingangs erläuterten Oberflächenmodifikationsmittel verwendet werden.

Eine weitere Möglichkeit zur Herstellung der nanopartikulären Teilchen besteht in dem sogenannten GAS-Verfahren (Gas Anti Solvent Recrystallization). Das GAS-Verfahren nutzt ein hochkomprimiertes Gas oder überkritisches Fluid (z.B.

Kohlendioxid) als Nicht-Lösungsmittel zur Kristallisation von gelösten Stoffen. Die verdichtete Gasphase wird in die Lösung des Wachses in einem geeigneten organischen Lösungsmittel, wie vorstehend beschrieben, eingeleitet und dort absorbiert. Dabei vergrößert sich das Flüssigkeitsvolumen, die Löslichkeit des Wachses nimmt ab und es wird in Form feinteiliger Partikel abgeschieden.

Weiter geeignet ist das PCA-Verfahren (Precipitation with a Compressed Fluid Anti-Solvent). Hier wird die Lösung des Wachses in einem geeigneten organischen Lösungsmittel, wie vorstehend beschrieben, in ein überkritisches Fluid, wie beispielsweise Kohlendioxid, eingedüst. Dabei bilden sich feinstverteilte Tröpfchen, in denen Diffusionsvorgänge ablaufen, die zu einer Ausfällung feinster Partikel führen.

Beim PGSS-Verfahren (Particles from Gas Saturated Solutions) wird das Wachs durch Aufpressen von Gas (z. B. Kohlendioxid oder Propan) aufgeschmolzen. Druck und Temperatur erreichen nahe- oder überkritische Werte. Die Gasphase löst sich im Feststoff und bewirkt eine Absenkung der Schmelztemperatur, der Viskosität und der Oberflächenspannung. Bei der Expansion durch eine Düse kommt es durch Abkühlungseffekte zur Bildung feiner Teilchen.

Beim RESS-Verfahren (Rapid expansion of supercritical solutions) wird das Wachs in einem Druckgefäß vorgelegt, der Reaktor verschlossen und das überkritische Lösungsmittel (CO₂, Ethylen, Propan, Ammoniak, Methanol, N₂O, N₂O₂, SF₆, Difluormethan, Trifluormethan, Wasser, Toluol, etc., bevorzugt jedoch CO₂) solange unter Temperaturerhöhung aufgepreßt, bis die gewünschten überkritischen Bedingungen erreicht sind. Zusammen mit dem Wachs kann auch ein Oberflächenmodifikationsmittel vorgelegt werden. Nachdem das Wachs, ggf. in Gegenwart des Oberflächenmodifikationsmittels, unter überkritischen Bedingungen gelöst wurde, wird die Lösung durch eine Düse expandiert, wobei es zur Bildung von Nanopartikeln kommt. Die Expansion kann in Luft, ein anderes Gas oder aber auch in eine Flüssigkeit, z.B. Wasser, hinein erfolgen, wobei diese Flüssigkeit wiederum ein Oberflächenmodifikationsmittel enthalten kann, um ein unerwünschtes Zusammenbacken der Partikel zu verhindern.

Beim Schmelz-Emulgier-Verfahren wird das Wachs in eine Flüssigkeit gebracht, in der es nicht löslich ist (Nicht-Lösungsmittel). Als Nicht-Lösungsmittel kommen Wasser oder Mischungen aus Wasser und nichtwässrigen polaren Lösungsmitteln, wie beispielsweise Ethanol, Glycerin, Ethylenglykol, Propylenglykol oder N-Methylpyrrolidon in Betracht. Als Nicht-Lösungsmittel bevorzugt ist Wasser. Anschließend wird das Gemisch über den Schmelzpunkt des Wachses unter Ausbildung eines Zweiphasensystems erhitzt. Durch Zugabe eines oder mehrerer Oberflächenmodifikationsmittel wird die flüssige Phase des geschmolzenen Wachses emulgiert, und zwar so, daß sehr feine Tröpfchen entstehen. Die Emulgierung erfolgt unter Einbringung von mechanischer Energie mittels eines herkömmlichen Rührers, beispielsweise eines Blatt- oder Ankerrührers. Nach der Bildung der Mikroemulsion wird diese unter den Schmelzpunkt des Wachses abgekühlt, wobei eine Nano-Dispersion des Wachses entsteht. Das Oberflächenmodifikationsmittel kann auch bereits zu Beginn des Verfahrens vorgelegt werden, bevor das Wachs geschmolzen wird. In einer weiteren Abwandlung des Verfahrens wird das Wachs aufgeschmolzen, ggf. in Gegenwart eines Oberflächenmodifikationsmittels. Davon getrennt wird das Nicht-Lösungsmittel auf eine Temperatur oberhalb des Schmelzpunkts des Wachses erwärmt, ggf. in Gegenwart eines Oberflächenmodifikationsmittels. Anschließend wird die Schmelze des Wachses, ggf. im Gemisch mit dem Oberflächenmodifikationsmittel, in das erwärmte Nicht-Lösungsmittel unter Rühren eingetropft und nach erfolgter Emulgierung unter den Schmelzpunkt des Wachses abgekühlt.

Bei der Herstellung von im Rahmen der vorliegenden Erfindung verwendeten nanopartikulären Wachs-Dispersionen wurde überraschenderweise gefunden, daß im Gegensatz zu der aus dem Stand der Technik bekannten Lehre, beispielsweise derjenigen des europäischen Patentes 0 506 197, eine intensive Durchmischung des Reaktionsgemischs während der Emulgierung des Wachses nicht erforderlich ist. Spezielle Vermischungstechniken wie die Verwendung von Hochgeschwindigkeitsrührern oder Hochdruckhomogenisatoren oder die Anwendung von Ultraschall sind demnach nach dem im Rahmen der vorliegenden Erfindung gefundenen Verfahren nicht erforderlich. Dies bedeutet für die technische Durchführung des Verfahrens einen erheblichen wirtschaftlichen Vorteil.

Im Rahmen der vorliegenden Erfindung wurde somit ein Verfahren zur Herstellung wäßriger Dispersionen von nanopartikulären Wachsen gefunden, wobei das Wachs einen Schmelzpunkt von mindestens 40°C hat und wobei der mittlere Teilchendurchmesser des Wachses in der Dispersion im Bereich von 5 bis 200 nm liegt, in welchem
(a) das geschmolzene Wachs in Gegenwart von Wasser mittels eines oder mehrerer Oberflächenmodifikationsmittel emulgiert wird,
(b) wobei die Emulgierung ohne intensive Durchmischung, insbesondere ohne Anwendung eines Hochgeschwindigkeitsrührers oder Hochdruckhomogenisators oder vergleichbarer Durchmischungsvorrichtungen erfolgt, und
(c) die Emulsion anschließend unter den Schmelzpunkt der emulgierten Wachströpfchen abgekühlt wird.

Als Oberflächenmodifikationsmittel eignen sich besonders nichtionische Emulgatoren, wobei Alkylpolyglycoside, insbesondere solche, deren Alkylrest 12 bis 16 Kohlenstoffatome enthält, bevorzugt sind.

Ein weiterer Gegenstand der Erfindung ist somit ein nanopartikuläres Wachs, wobei das Wachs einen Schmelzpunkt von mindestens 40°C hat und wobei der mittlere Teilchendurchmesser des Wachses im Bereich von 5 bis 200 nm liegt, welches herstellbar ist durch
(a) Emulgierung des geschmolzenen Wachses in Gegenwart von Wasser mittels eines oder mehrerer Oberflächenmodifikationsmittel, ohne daß die Emulgierung unter intensiver Durchmischung erfolgt, insbesondere ohne daß ein Hochgeschwindigkeitsrührer oder Hochdruckhomogenisator verwendet wird, und
(b) Abkühlung der Emulsion unter den Schmelzpunkt der emulgierten Wachströpfchen.

Die nach dem erfindungsgemäßen Verfahren hergestellten wäßrigen Dispersionen nanopartikulärer Wachse zeichnen sich durch eine hohe Lagerstabilität aus.

Aus der EP-A2 0 786 251 ist beispielsweise ein Verfahren zur Herstellung einer Dispersion von hydriertem Rizinusöl bekannt, in welchem hydriertes Rizinusöl mit Wasser in Gegenwart von Natrium-Laurylsulfat unter Anwendung eines Hochdruckhomogenisators dispergiert wird. Nach dem Abkühlen der gebildeten Dispersion wird zunächst eine Dispersion mit einer mittleren Teilchengröße von 195 nm erhalten, die sich nach 24 Stunden zu einem Gel verfestigte. Für kosmetische Verwendungen im Sinne der vorliegenden Erfindung ist jedoch ein derartiges Gel nicht bzw. nur eingeschränkt einsetzbar. Nach dem Verfahren der vorliegenden Erfindung wurde mit Alkylpolyglycosid als Emulgator und unter Anwendung eines einfachen Blattrührers eine Dispersion von hydriertem Rizinusöl mit einer mittleren Teilchengröße von 70 nm erhalten, welche sich auch nach 6 Monaten Lagerung nicht verändert hatte. Im Lichte des vorgenannten Stands der Technik war es überraschend, daß durch die zunächst als geringfügig erscheinenden Abwandlungen, welche in dem Verfahren gemäß der vorliegenden Erfindung vorgenommen worden waren, die Nachteile des Stands der Technik überwunden werden konnten. Besonders überraschend war es dabei, daß durch eine Vereinfachung der bekannten Lehre, nämlich durch das erfindungsgemäße Ersetzen der Hochdruckhomogenisation durch einfache Rührverfahren verbunden mit dem Eintrag einer deutlich geringeren Rührenergie, bessere Ergebnisse erzielt werden konnten. Diese besseren Ergebnisse bestehen insbesondere in einer erhöhten Lagerstabilität der nanopartikulären Dispersion sowie einer Verminderung der Teilchengröße des nanopartikulären Wachses.

Als weiteres Verfahren zur Herstellung nanopartikulärer Wachse ist das Mahlverfahren zu nennen. Beim Mahlverfahren wird das Wachs durch Mahlen, beispielsweise in einer Schwingmühle, Perlmühle oder Rührwerkskugelmühle zerkleinert, wobei Mahlkörper mit einem mittleren Durchmesser unterhalb von 200 µm verwendet werden. Ggf. erfolgt das Mahlen in Gegenwart eines Oberflächenmodifikationsmittels. Der Mahlvorgang kann trocken oder aber in einem Nicht-Lösungsmittel für das Wachs erfolgen. Der Mahlvorgang kann auch unter Kühlung erfolgen.

Nanopartikel, die bei der Herstellung als Suspension in Wasser oder einer anderen flüssigen Phase anfallen, können durch übliche Verfahren aufkonzentriert bzw. als Feststoff isoliert werden, z. B. durch eine Sprühtrocknung, Gefriertrocknung, oder Ultrafiltration.

Bevorzugt erfolgt die Herstellung der erfindungsgemäß verwendeten nanopartikulären Wachsteilchen nach dem Schmelz-Emulgier-Verfahren oder dem Evaporationsverfahren. Vorzugsweise liegen die Nanopartikel am Ende des Herstellprozesses in Form einer wässrigen oder wasserhaltigen Suspension vor.

Das in der erfindungsgemäßen Verwendung eingesetzte Wachs soll zusammen mit dem verwendeten Oberflächenmodifikationsmittel in der Lage sein, gemäß zumindest einem der vorstehend beschriebenen Herstellverfahren in Wasser stabil dispergierbare Nanopartikel zu ergeben, wobei der mittlere Teilchendurchmesser der Nanopartikel im Bereich von 5 bis 200 nm liegt. Die brauchbaren Wachse und Kombinationen von Wachsen und Oberflächenmodifikationsmitteln lassen sich durch einfache Routineversuche auswählen.

Im Sinne der Erfindung besonders bevorzugt ist die Verwendung von wie eingangs definierten nanopartikulären Wachsen, welche nach einem Verfahren hergestellt werden, in dem
(a) das geschmolzene Wachs in Gegenwart von Wasser mittels eines oder mehrerer Oberflächenmodifikationsmittel emulgiert wird, wobei ganz besonders bevorzugt dabei die Emulgierung ohne intensive Durchmischung, insbesondere ohne Anwendung eines Hochgeschwindigkeitsrührers oder Hochdruckhomogenisators erfolgt, und
(b) die Emulsion unter den Schmelzpunkt der emulgierten Wachströpfchen abgekühlt wird.

Die aufgeführten Herstellverfahren sind lediglich beispielhaft zu verstehen und stellen keine Einschränkung dar.

Nach der Lehre der vorliegenden Erfindung wird ein nanopartikuläres Wachs, welches einen Schmelzpunkt von mindestens 40°C und einen mittleren Teilchendurchmesser im Bereich von 5 bis 200 nm aufweist, in einer Zubereitung, vorzugsweise einer Zubereitung zur Pflege von Keratinfasern, insbesondere menschlichen Haaren, eingesetzt.

Die erfindungsgemäß verwendeten Zubereitungen können als Lotion, Emulsion, Mikroemulsion, Lösung, Creme oder Gel formuliert sein. Als kosmetische Mittel können erfindungsgemäß alle Zubereitungen verwendet werden, die in Form von wäßrigen Zubereitungen auf dem Haar zur Anwendung kommen. Solche Zubereitungen sind z.B. Haarspülungen, Haarshampoos, Haarfärbepräparate (oxidative und direktziehende), Dauerwellen, Haarkurpräparate und andere Haarkosmetika. Besonders bevorzugt sind Haarspülungen und Haarshampoos. Ebenfalls bevorzugt ist die Verwendung in Haarnachbehandlungsmitteln, d. h. Mitteln, die nach einer Haarwäsche oder einer anderen Haarbehandlung, wie Dauerwellen oder Färbung, eingesetzt werden.

Erfindungsgemäß weiterhin bevorzugt sind schäumende Zubereitungen.

Die erfindungsgemäß verwendetenen Zubereitungen können in Form von "leave-on"-Produkten, die nach der Anwendung auf dem Haar verbleiben, wie Lotionen, Sprays oder Frisierschäumen, in Form von Lotionen, Sprays oder Schäumen zum Legen einer Wasserwelle oder in Form von Lotionen, Sprays oder Schäumen zum Fönen vorliegen, die nach Verfahren hergestellt werden können, welche dem Fachmann geläufig sind. Die erfindungsgemäßen Zubereitungen können auch als "rinse-off"-Produkte vorliegen, die nach der Anwendung nicht auf dem Haar verbleiben, wie Shampoos, Lotionen, Sprays oder Schäume, und sie können Behandlungsprodukte darstellen, die vor oder nach dem Färben oder Entfärben, vor oder nach dem Shampoonieren oder vor oder nach dem Legen einer Dauerwelle angewandt werden.

Als weitere Rezepturbestandteile können in den Zubereitungen ein oder mehrere Adjuvantien enthalten sein, wie sie üblicherweise in solchen Zubereitungen verwendet werden, wie z. B. Konservierungsmittel, Bakterizide, Antioxidantien, Parfüme, Schaumbremsen, Farbstoffe, Pigmente, Verdickungsmittel, anfeuchtende und/oder feuchthaltende Substanzen, Tenside, Emulgatoren, Weichmacher, Fette, Öle, Wachse, Silikone, Sequestrierungsmittel, anionische, kationische, nichtionische oder amphotere Polymere, Alkalinisierungs- oder Acidifizierungsmittel, Alkohole, Polyole, Enthärter, Lichtschutzmittel, Elektrolyte und organische Lösungsmittel. Einzelne dieser Stoffe, wie z. B. Emulgatoren, können in den erfindungsgemäßen Zubereitungen einerseits als Adjuvans, unabhängig davon aber auch zusätzlich als Oberflächenmodifikationsmittel der Nanopartikel enthalten sein.

Als Adjuvantien kommen beispielsweise in Betracht
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle
- Lösungsvermittler wie Ethanol, Isopropanol, Ethylenglykol, Propylenglykol Glycerin und Diethylenglykol,
- Farbstoffe zum Einfärben der Zubereitungen,
- Antischuppenwirkstoffe wie Piroctone Olamine und Zink Omadine, Salicylsäure
- weitere Substanzen zur Einstellung des pH-Wertes,
- Wirkstoffe wie Panthenol, Pantothensäure, Niacinamid, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Guanidine, Harnstoffe sowie primäre, sekundäre und tertiäre Phosphate,
- Trübungsmittel wie Latex,
- Perlglanzmittel wie Ethylenglykolmono- und -distearat,
- Schaumstabilisatoren
- Treibmittel wie Propan-Butan-Gemische, N₂O, Dimethylether, CO₂ und Luft,
- Antioxidantien
- Konservierungsmittel wie z.B. p-Hydroxybenzoesäureester, Phenoxyethanol, Na-Benzoat
- Tenside sowie
- kationische Polymere.

Zur Herstellung der vorstehend genannten Zubereitungen wird vorzugsweise eine wäßrige oder wasserhaltige Suspension der Wachs-Nanopartikel mit den weiteren Rezepturbestandteilen in an sich bekannter Weise bei einer Temperatur unterhalb des Schmelzpunkts der Nanopartikel vermischt bzw. dispergiert.

Die Einsatzmenge des nanopartikulären Wachses in der Zubereitung wird so gewählt, daß die Konzentration des in den Nanopartikeln enthaltenen Wachses - d. h. ohne Berücksichtigung ggf. zusätzlich in den Nanopartikeln enthaltener Oberflächenmodifikationsmittel - zwischen 0,01 und 10, vorzugsweise zwischen 0,05 und 5, und besonders bevorzugt zwischen 0,1 und 1 Gew.-% bezogen auf die Zubereitungen liegt.

Das nanopartikuläre Wachs bewirkt bereits in kleinen Mengen auf der Keratinfaser eine Pflege im Sinne von konditionierenden Effekten, worunter im Rahmen der vorliegenden Erfindung insbesondere eine Erhöhung der Naßkämmbarkeit der Keratinfasern sowie ein nachhaltig verbesserter Griff der Keratinfasern zu verstehen ist. Mit Griff wird das taktile Empfinden beim Greifen mit der Hand in die Keratinfasern, insbesondere die Haare, beschrieben. Das nanopartikuläre Wachs verleiht den Keratinfasern darüber hinaus eine Trockenkämmbarkeit, welche im Fall der Anwendung an menschlichen Haaren in einem für den Halt der Frisur sehr günstigen Bereich liegt, ohne daß in nennenswertem Umfang eine elektrostatische Aufladung und damit ein "Fliegen" der Haare beobachtet wird. Zusätzlich schützt das nanopartikuläre Wachs das Haar vor Schäden durch chemische Behandlungen wie beispielsweise Blondieren, Färben und Dauerwellen.

Es ist von besonderem Vorteil, daß die vorstehend beschriebenen, im Sinne der Erfindung erwünschten Wirkungen der erfindungsgemäß verwendeten nanopartikulären Wachse bereits bei niedrigen Einsatzkonzentrationen der Wachse auftreten. Somit ist in den Zubereitungen eine sparsame Dosierung der nanopartikulären Wachse möglich.

Ebenfalls Gegenstand der vorliegenden Erfindung ist ein Verfahren zur pflegenden Behandlung von Keratinfasern, insbesondere menschlichen Haaren, bei welchem eine wie vorstehend definierte Zubereitung in einer wirksamen Menge auf die Keratinfasern aufgebracht wird.

Unter der pflegenden Behandlung ist vorzugsweise eine Konditionierung zu verstehen, insbesondere eine Erhöhung der Naßkämmbarkeit und eine Verbesserung des Griffs.
Die erfindungsgemäß verwendeten nanopartikulären Wachse üben neben ihren konditionierenden Wirkungen auf Keratinfasern zusätzlich Effekte auf die menschliche Haut aus. Bei Einwirkung auf die Haut bewirken sie eine Verminderung von deren Wasserverlust und damit eine Erhöhung der Hautfeuchtigkeit. Weiterhin bewirken sie eine Rückfettung der Haut, eine Verbesserung von deren Barriereeigenschaften und eine Verbesserung des Hautgefühls. Darüber hinaus wurde gefunden, daß die im Sinne der vorliegenden Erfindung definierten nanopartikulären Wachse die Hautverträglichkeit kosmetischer Zusammensetzungen verbessern, insbesondere von Zusammensetzungen zur Reinigung des Körpers. Unter der Hautverträglichkeit einer Zusammensetzung ist dabei insbesondere deren Einfluß auf die Hautreaktionen Erythem, Ödem, Schuppung und Fissur bei Applikation der Zusammensetzung auf die Haut zu verstehen.

Die erfindungsgemäß verwendeten nanopartikulären Wachse bewirken somit eine Pflegewirkung nicht nur auf die Haare, sondern auch auf die Haut. Damit sind sie besonders vorteilhaft zur Verwendung in haarkosmetischen Zubereitungen geeignet, da bei deren Anwendung stets nicht nur die Haare in Kontakt mit diesen Zubereitungen kommen, sondern stets auch die Haut, insbesondere die Kopfhaut und die Haut der Hände.

Unter der Pflege der Haut ist dabei insbesondere eine Rückfettung und eine Verstärkung der Barrierewirkung der Haut zu verstehen.

Die erfindungsgemäßen Wachs-Nanopartikel ermöglichen darüber hinaus die Herstellung von Zusammensetzungen mit einem höheren Wachsanteil, als es mit den aus dem Stand der Technik bekannten Wachsformen möglich ist. Insbesondere ermöglichen sie die Herstellung von schäumenden Formulierungen, die einerseits die gewünschten Effekte, wie z. B. eine gute konditionierende Wirkung, aufweisen und gleichzeitig eine gute Schaumbildung zeigen.

Als weiterer Vorteil kommt hinzu, daß sich die erfindungsgemäßen nanopartikulären Wachse stabil in Formulierungen, selbst wenn sie einen hohen Wasseranteil enthalten, einarbeiten lassen, ohne daß diese instabil werden und ohne daß es zu einer Phasentrennung, Sedimentation oder Teilchengrößenzunahme der Wachspartikel kommt.

Die folgenden Beispiele sollen die vorliegende Erfindung verdeutlichen, ohne sie einzuschränken. Alle Prozentanteile sind, soweit nicht anders angegeben, auf das Gesamtgewicht der Zubereitungen bezogen.

### Beispiele:

### Beispiele 1.1-1.2: Herstellung von Dispersionen nanopartikulärer Wachse

### Beispiel 1.1: Herstellung einer Dispersion von nanopartikulärem hydrierten Rizinusöl

Zu einer auf 98°C erwärmten Schmelze von 3,0 kg Cutina HR (hydriertes Rizinusöl, Warenzeichen der Cognis Deutschland GmbH) wurde eine auf 95°C erwärmte Lösung von 6,0 kg Plantacare 1200 UP (Konservierungsmittel-freies C12-C16-Alkylglycosid mit 51% Aktivsubstanz, Warenzeichen der Cognis Deutschland GmbH) in 21,0 kg dest. Wasser unter Rühren zugegeben. Anschließend wurde das Gemisch eine Stunde bei 95°C nachgerührt, mit 30 g Euxyl K 400 (Methyldibromglutaronitril und Phenoxyethanol, Konservierungsmittel) versetzt und unter Rühren auf Raumtemperatur abgekühlt. Das Rühren erfolgte jeweils mit einem Blattrührer bzw. einem Ankerrührer und ca. 50-100 Umdrehungen pro Minute. Es wurden 30,0 kg einer lagerstabilen milchig-weißen Dispersion erhalten, welche nach Bestimmung mittels eines Transmissionselektronenmikroskops Teilchengrößen zwischen 30 und 500 nm aufwies. Bei der Bestimmung der Teilchengröße durch die Laser-Back-Scattering-Methode mittels eines Ultrafine Particle Analyzers (UPA 3.150, Fa. Grimm, Ainring) ergab sich eine mittlere Teilchengröße von 70 nm.

### Beispiel 1.2: Herstellung einer Dispersion von nanopartikulärem Cetylstearylalkohol (nicht erfindungsgemäß)

3,0 kg Lanette ○ (Cetylstearylalkohol, Warenzeichen der Cognis Deutschland GmbH) wurden bei 98°C mit 3,0 kg Texapon N 70 (Natriumlaurylethersulfat mit 2 EO, 70% Aktivsubstanz, Warenzeichen der Cognis Deutschland GmbH) aufgeschmolzen. Die Schmelze wurde zu 24,0 kg auf 98°C vorgeheiztes Wasser unter Rühren zugegeben. Anschließend wurde das Gemisch eine Stunde bei 98°C nachgerührt, mit 30 g Euxyl K 400 (Methyldibromglutaronitril und Phenoxyethanol, Konservierungsmittel) versetzt und unter Rühren auf Raumtemperatur abgekühlt.

Das Rühren erfolgte jeweils mit einem Blattrührer bzw. einem Ankerrührer und ca. 50-100 Umdrehungen pro Minute. Es wurden 30,0 kg einer lagerstabilen milchig-weißen Dispersion erhalten, welche nach Bestimmung mittels eines Ultrafine Particle Analyzers (UPA 3.150) eine mittlere Teilchengröße von 105 nm aufwies.

### Beispiele 2.1 bis 2.5: Haarshampoos

| | Beispiel 2.1 | Beispiel 2.2 (nicht erfindungsgemäß) |
|---|---|---|
| Rezepturkomponente | Gew.-% | Gew.-% |
| Natriumlaurethsulfat | 18 | 18 |
| Cocoamidopropylbetain | 4,0 | 4,0 |
| Dispersion aus Beispiel 1.1 | 3,0 | 0 |
| Dispersion aus Beispiel 1.2 | 0 | 3,0 |
| Propylenglycol | 4,0 | 4,0 |
| Imidazolidinyl Harnstoff | 0,3 | 0,3 |
| PEG-7 Glyceryl Cocoat | 2,0 | 2,0 |
| Parfüm | 0,07 | 0,07 |
| lodopropinyl Butylcarbamat | 0,05 | 0,05 |
| Kochsalz | 1,0 | 1,0 |
| Wasser | ad 100 | ad 100 |

| | Beispiel 2.3 (Vergleichsbeispiel) | Beispiel 2.4 (nicht erfindungsgemäß) | Beispiel 2.5 (nicht erfindungsgemäß) |
|---|---|---|---|
| Rezepturkomponente | Gew.-% | Gew.-% | Gew.-% |
| Texapon NSO | 43 | 43 | 43 |
| Tego Betain F 50 | 7,0 | 7,0 | 7,0 |
| Dispersion aus Beispiel 1.2 | 0 | 2,0 | 5,0 |
| Natriumsalicylat | 0,53 | 0,53 | 0,53 |
| Wasser | ad 100 | ad 100 | Ad 100 |
| pH-Wert der Rezeptur | 4,77 | 4,83 | 4,86 |

Die Produktleistung der Haarshampoos aus den Beispielen 2.3 bis 2.5 wurde an Haarsträhnen geprüft, wobei die in der nachfolgenden Tabelle aufgeführten Parameter nach einem Schulnotensystem (1 = sehr gut, bis 6 = sehr schlecht) bewertet wurden.

| | Beispiel 2.3 (Vergleichsbeispiel) | Beispiel 2.4 | Beispiel 2.5 |
|---|---|---|---|
| Parameter | Bewertung | Bewertung | Bewertung |
| Naßkämmbarkeit | 5 - | 3 - | 4 |
| Griff ins nasse Haar | 5 | 4 + | 4 |
| Trockenkämmbarkeit | 2 | 2 + | 2 + |
| Griff ins trockene Haar | 3 + | 2 | 2 + |
| Statische Aufladung | Sehr stark | leicht | sehr stark |
| Belastung | Keine | keine | Keine |
| Glanz | 3 | 3 | 3 |

Das nicht erfindungsgemäße Beispiel 2.4 zeigt im Vergleich zum Vergleichsbeispiel 2.3 eine deutliche Verbesserung in der Naßkämmbarkeit und im Griff ins nasse Haar sowie eine leichte Verbesserung bezüglich der Trockenkämmbarkeit und des Griffs ins trockene Haar. Das nicht erfindungsgemäße Beispiel 2.5 zeigt im Vergleich zum Vergleichsbeispiel 2.3 eine leichte Verbesserung in allen Parametern (Naßkämmbarkeit, Griff nass, Trockenkämmbarkeit und Griff trocken) und somit wie Beispiel 2.4 eine verbesserte Konditionierwirkung.

### Beispiele 3.1 und 3.2: Haarkonditionierungsmittel

| | Beispiel 3.1 | Beispiel 3.2 (nicht erfindungsgemäß) |
|---|---|---|
| Rezepturkomponente | Gew.-% | Gew.-% |
| Cyclomethicon, Dimethiconol, Dimethicon | 27 | 27 |
| Phenyl-Trimethicon | 4,5 | 4,5 |
| Dispersion aus Beispiel 1.1 | 2,0 | 0 |
| Dispersion aus Beispiel 1.2 | 0 | 2,0 |
| Propylenglycol | 29 | 29 |
| Methylchloroisothiazolinon, Methylisothiazolinon | 0,05 | 0,05 |
| Parfüm | 0,3 | 0,3 |
| Farbstoff | 0,25 | 0,25 |
| Polyacrylamid, C13-14-Isoparaffin, Laureth-7 | 2,55 | 2,55 |
| Wasser | ad 100 | ad 100 |

## Patentansprüche

1. Nanopartikuläres Wachs, wobei das Wachs einen Schmelzpunkt von mindestens 40°C hat, der mittlere Teilchendurchmesser des Wachses im Bereich von 5 bis 200 nm liegt und das nanopartikuläre Wachs von mindestens einem Oberflächenmodifikationsmittel, das ein Alkylpolyglycosid ist, umhüllt ist, **dadurch gekennzeichnet, dass** für die Herstellung des nanopartikulären Wachses das Wachs und das Oberflächenmodifikationsmittel im Gewichtsverhältnis 1 : 2 bis 2: 1 eingesetzt werden, ausgenommen:
a) ein nanoskaliges Wachs mit einer Partikelgröße von 70 nm, hergestellt aus 10 Gewichtsteilen Lanolin, 10 Gewichtsteilen des Handelsproduktes Texapon N 70 (entsprechend 6,8 - 7,3 Gewichtsteilen Natriumlaureth-2-sultat) und 10 Gewichtsteilen des Handelsproduktes Plantacare 818 (entsprechend 5,1 - 5,3 Gewichtsteilen Coco Glucosides);
b) ein nanoskaliges Wachs mit einer Partikelgröße von 100 nm, hergestellt aus 15 Gewichtsteilen Lanolin und 15 Gewichtsteilen des Handelsproduktes Plantacare 818 (entsprechend 7,65 - 7,95 Gewichtsteilen Coco Glucosides).

2. Nanopartikuläres Wachs gemäß Anspruch 1, **dadurch gekennzeichnet, dass** das Wachs ausgewählt ist aus der Gruppe, die gebildet wird von natürlichen Wachsen, chemisch modifizierten Wachsen, synthetischen Wachsen, Fettalkoholen mit 14 bis 40 Kohlenstoffatomen und Wachsestern mit 20 bis 44 Kohlenstoffatomen.

3. Nanopartikuläres Wachs gemäß Anspruch 1 oder 2, **dadurch gekennzeichnet, dass** das Wachs gehärtetes Rizinusöl oder Cetylstearylalkohol ist.

4. Nanopartikuläres Wachs gemäß Anspruch 3, **dadurch gekennzeichnet, dass** ein Gemisch aus zwei oder mehr Obarflächenmodifikationsmitteln eingesetzt wird.

5. Nanopartikuläres Wachs gemäß einem der Ansprüche 1 - 4, **dadurch gekennzeichnet, dass** es in Form einer wässrigen oder wasserhaltigen Suspension vorliegt.

6. Nanopartikuläres Wachs gemäß einem der Ansprüche 1 - 5, **dadurch gekennzeichnet, dass** der Alkylrest des Alkylpolyglycosids zwischen 12 und 16 Kohlenstoffatome enthält.

7. Kosmetische Zubereitung, enthaltend ein oder mehrere übliche Adjuvantien, ausgewählt aus Konservierungsmitteln, Bakteriziden, Antioxidantien, Parfüms, Schaumbremsen, Farbstoffen, Pigmenten, Verdickungsmitteln, anfeuchtenden und/oder feuchthaltenden Substanzen, Tensiden, Emulgatoren, Weichmachern, Fetten, Ölen, Wachsen, Silikonen, Sequestrierungsmitteln, anionischen, kationischen, nichtionischen oder amphoteren Polymeren, Alkalinisierungs- oder Acidifizierungsmitteln, Alkoholen, Polyolen, Enthärtern, Lichtschutzmitteln, Elektrolyten und organischen Lösungsmitteln, **dadurch gekennzeichnet, dass** ein nanopartikuläres Wachs gemäß einem der Ansprüche 1 - 6 enthalten ist.

8. Kosmetische Zubereitung gemäß Anspruch 7, **dadurch gekennzeichnet, dass** das nanopartikuläre Wachs in der Zubereitung in einer Konzentration von 0,01 bis 10 und vorzugsweise von 0,1 bis 5 Gew.-%, bezogen auf das Gesamtgewicht der Zubereitung, enthalten ist.

9. Kosmetische Zubereitung gemäß Anspruch 7 oder 8, **dadurch gekennzeichnet, dass** die Zubereitung als Lotion, Emulsion, Mikroemulsion, Lösung, Creme oder Gel formuliert ist.

10. Kosmetische Zubereitung gemäß einem der Ansprüche 7-9, **dadurch gekennzeichnet, dass** die Zubereitung als Haarspülung, Haarshampoo, oxidatives Haarfärbepräparat, direktziehendes Haarfärbepräparat, Dauerwelle, Haarkurpräparat oder Haarnachbehandlungsmittel formuliert ist.

11. Nicht-therapeutische, kosmetische Verwendung eines nanopartikulären Wachses gemäß einem der Ansprüche 1-6 oder einer kosmetischen Zubereitung gemäß einem der Ansprüche 7 - 10 zur Behandlung und/oder Pflege von Keratinfasern, insbesondere menschlichen Haaren.

12. Nicht-therapeutische, kosmetische Verwendung gemäß Anspruch 11 zur Konditionierung und/oder zur Erhöhung der Nasskämmbarkeit und/oder zur Verbesserung des Griffs von Keratinfasern, insbesondere menschlichen Haaren.

13. Nicht-therapeutische, kosmetische Verwendung gemäß Anspruch 11 oder 12 zum Schutz des Haars vor Schäden durch chemische Behandlungen wie Blondieren, Färben und Dauerwellen.

14. Nicht-therapeutisches, kosmetisches Verfahren zur pflegenden Behandlung von Keratinfasern, insbesondere menschlichen Haaren, **dadurch gekennzeichnet, dass** ein nanopartikuläres Wachs gemäß einem der Ansprüche 1 - 6 oder eine kosmetische Zubereitung gemäß einem der Ansprüche 7 - 10 in einer wirksamen Menge auf die Keratinfasern aufgebracht wird.

## Claims

1. Nanoparticulate wax, where the wax has a melting point of at least 40°C, the average particle diameter of the wax is in the range from 5 to 200 nm and the nanoparticulate wax is surrounded by at least one surface modifier, which is an alkyl polyglycoside, **characterized in that**, for the preparation of the nanoparticulate wax, the wax and the surface modifier are used in the weight ratio 1:2 to 2:1, with the exception of:
a) a nanoscale wax with a particle size of 70 nm, prepared from 10 parts by weight of lanolin, 10 parts by weight of the commercial product Texapon N 70 (corresponding to 6,8 - 7,3 parts by weight of sodium laureth-2 sulfate) and 10 parts by weight of the commercial product Plantacare 818 (corresponding to 5,1 - 5,3 parts by weight of Coco Glucosides),
b) a nanoscale wax with a particle size of 100 nm, prepared from 15 parts by weight of lanolin and 15 parts by weight of the commercial product Plantacare 818 (corresponding to 7,65 - 7,95 % by weight of Coco Glucosides).

2. Nanoparticulate wax according to Claim 1, **characterized in that** the wax is selected from the group which is formed by natural waxes, chemically modified waxes, synthetic waxes, fatty alcohols having 14 to 40 carbon atoms and wax esters having 20 to 44 carbon atoms.

3. Nanoparticulate wax according to Claim 1 or 2, **characterized in that** the wax is hydrogenated castor oil or cetylstearyl alcohol.

4. Nanoparticulate wax according to Claim 3, **characterized in that** a mixture of two or more surface modifiers is used.

5. Nanoparticulate wax according to one of Claims 1-4, **characterized in that** it is in the form of an aqueous or water-containing suspension.

6. Nanoparticulate wax according to one of Claims 1-5, **characterized in that** the alkyl radical of the alkyl polyglycoside contains between 12 and 16 carbon atoms.

7. Cosmetic preparation comprising one or more customary adjuvants selected from preservatives, bactericides, antioxidants, perfumes, antifoams, dyes, pigments, thickeners, moisturizing and/or humectant substances, surfactants, emulsifiers, emollients, fats, oils, waxes, silicones, sequestrants, anionic, cationic, nonionic or amphoteric polymers, alkalinizing or acidifying agents, alcohols, polyols, softeners, photo-protective agents, electrolytes and organic solvents, **characterized in that** a nanoparticulate wax according to one of Claims 1-6 is present.

8. Cosmetic preparation according to Claim 7, **characterized in that** the nanoparticulate wax is present in the preparation in a concentration of from 0.01 to 10% by weight and preferably from 0.1 to 5% by weight, based on the total weight of the preparation.

9. Cosmetic preparation according to Claim 7 or 8, **characterized in that** the preparation is formulated as a lotion, emulsion, microemulsion, solution, cream or gel.

10. Cosmetic preparation according to one of Claims 7-9, **characterized in that** the preparation is formulated as a hair rinse, hair shampoo, oxidative hair colouring preparation, direct hair colouring preparation, permanent wave, hair treatment preparation or hair after-treatment composition.

11. Nontherapeutic, cosmetic use of a nanoparticulate wax according to one of Claims 1-6 or a cosmetic preparation according to one of Claims 7-10 for the treatment and/or care of keratin fibres, in particular human hair.

12. Nontherapeutic, cosmetic use according to Claim 11 for the conditioning and/or increasing the wet combability and/or for improving the feel of keratin fibres, in particular human hair.

13. Nontherapeutic, cosmetic use according to Claim 11 or 12 for protecting the hair against damage by chemical treatments such as bleaching, colouring and permanent waving.

14. Nontherapeutic, cosmetic method for the caring treatment of keratin fibres, in particular human hair, **characterized in that** a nanoparticulate wax according to one of Claims 1-6 or a cosmetic preparation according to one of Claims 7-10 is applied to the keratin fibres in an effective amount.

## Revendications

1. Cire nanoparticulaire, la cire possédant un point de fusion d'au moins 40 °C, le diamètre moyen de particule de la cire se situant dans la plage de 5 à 200 nm et la cire nanoparticulaire étant enveloppée d'au moins un agent de modification de la surface qui représente un alkylpolyglycoside, **caractérisée en ce que** l'on met en oeuvre, pour la préparation de la cire nanoparticulaire, la cire et l'agent de modification de la surface dans le rapport pondéral de 1 : 2 à 2 : 1, exempt d'
a) une nanocire d'une grandeur particulaire de 70 nm, préparée de 10 parts en poids de lanoline, 10 parts en poids de produit commercial Texapon N 70 (correspondant à 6,8 - 7,3 parts en poids de laureth-2 sulfate de sodium) et 10 parts en poids de produit commercial Plantacare 818 (correspondant à 5,1 - 5,3 parts en poids de Coco Glucosides);
b) une nanocire d'une grandeur particulaire de 100 nm, preparée de 15 parts en poids de lanoline et 15 parts en poids de produit commercial Plantacare 818 (correspondant à 7,65 - 7,95 parts en poids de Coco Glucosides).

2. Cire nanoparticulaire selon la revendication 1, **caractérisée en ce que** la cire est choisie parmi le groupe qui est formé par des cires naturelles, des cires ayant subi une modification chimique, des cires synthétiques, des alcools gras contenant de 14 à 40 atomes de carbone et des esters de cires contenant de 20 à 44 atomes de carbone.

3. Cire nanoparticulaire selon la revendication 1 ou 2, **caractérisée en ce que** la cire représente de l'huile de ricin durcie ou de l'alcool cétylstéarylique.

4. Cire nanoparticulaire selon la revendication 3, **caractérisée en ce que** l'on met en oeuvre un mélange de deux agents de modification de la surface ou plus.

5. Cire nanoparticulaire selon l'une quelconque des revendications 1 à 4, **caractérisée en ce qu'**elle est présente sous la forme d'une suspension aqueuse ou contenant de l'eau.

6. Cire nanoparticulaire selon l'une quelconque des revendications 1 à 5, **caractérisée en ce que** le radical alkyle de l'alkylpolyglycoside contient entre 12 et 16 atomes de carbone.

7. Préparation cosmétique, contenant un ou plusieurs adjuvants habituels choisis parmi des conservateurs, des bactéricides, des antioxydants, des parfums, des inhibiteurs de mousse, des colorants, des pigments, des épaississants, des substances humidifiantes et/ou conservant l'humidité, des agents tensioactifs, des émulsifiants, des plastifiants, des graisses, des huiles, des cires, des silicones, des agents de séquestration, des polymères anioniques, cationiques, non ioniques ou amphotères, des agents d'alcalinisation ou d'acidification, des alcools, des polyols, des adoucissants, des agents de protection contre l'effet de la lumière, des électrolytes et des solvants organiques, **caractérisée en ce qu'**elle contient une cire nanoparticulaire selon l'une quelconque des revendications 1 à 6.

8. Préparation cosmétique selon la revendication 7, **caractérisée en ce que** la cire nanoparticulaire est contenue dans la préparation en une concentration de 0,01 à 10 et de préférence de 0,1 à 5 % en poids, rapportés au poids total de la préparation.

9. Préparation cosmétique selon la revendication 7 ou 8, **caractérisée en ce que** la préparation est formulée sous la forme d'une lotion, d'une émulsion, d'une microémulsion, d'une solution, d'une crème ou d'un gel.

10. Préparation cosmétique selon l'une quelconque des revendications 7 à 9, **caractérisée en ce que** la préparation est formulée sous la forme d'un rinçage capillaire, d'un shampooing capillaire, d'une préparation de teinture capillaire par oxydation, d'une préparation de teinture capillaire montant directement sur la fibre, d'une permanente, d'une préparation curative pour les cheveux ou d'un agent pour le traitement ultérieur des cheveux.

11. Utilisation cosmétique non thérapeutique d'une cire nanoparticulaire selon l'une quelconque des revendications 1 à 6, ou d'une préparation cosmétique selon l'une quelconque des revendications 7 à 10, pour le traitement et/ou les soins de fibres kératiniques, en particulier de cheveux humains.

12. Utilisation cosmétique non thérapeutique selon la revendication 11 pour le conditionnement et/ou pour augmenter l'aptitude du peignage au mouillé et/ou pour améliorer la tenue de fibres kératiniques, en particulier de cheveux humains.

13. Utilisation cosmétique non thérapeutique selon la revendication 11 ou 12 pour la protection des cheveux contre des dégradations dues à des traitements chimiques comme la décoloration, la teinture et le permanentage.

14. Procédé cosmétique non thérapeutique pour le traitement d'entretien de fibres kératiniques, en particulier de cheveux humains, **caractérisé en ce qu'**on applique une cire nanoparticulaire selon l'une quelconque des revendications 1 à 6 ou une préparation cosmétique selon l'une quelconque des revendications 7 à 10, en une quantité efficace, sur les fibres kératiniques.
